# EUROPEAN PATENT SPECIFICATION

(11) **EP 1 195 162 B1**
(45) Date of publication and mention of the grant of the patent: **04.06.2008**
(21) Application number: 00906681.2
(22) Date of filing: 03.03.2000
(51) Int. Cl.: A61K 39/39, A61K 39/145, A61K 39/15, A61K 39/165, A61K 39/21, A61K 39/10, A61K 39/05, A61K 39/106, A61K 39/108, A61K 39/118, A61K 39/00, A61K 39/012, A61K 39/015, A61P 31/14, A61P 31/16, A61P 31/18, A61P 31/20, A61P 31/04, A61P 33/06, A61P 33/02, A61P 33/12

(54) **VACCINE PREPARATION CONTAINING FATTY ACID AS A CONSTITUENT**
IMPFSTOFF-PRÄPARATION DIE EINE FETTSÄURE ALS INHALTSTOFF ENTHÄLT
PREPARATION DE VACCIN CONTENANT UN ACIDE GRAS COMME COMPOSANT

(30) Priority: 03.03.1999 JP 5573299
(43) Date of publication of application: 10.04.2002
(73) Proprietor: THE KITASATO INSTITUTE, Tokyo 108-8642 (JP)
(72) Inventor: YAMADA, Haruki, Minato-ku, Tokyo 108-8642 (JP); KIYOHARA, Hiroaki, Minato-ku, Tokyo 108-8642 (JP); NAGAI, Takayuki, Minato-ku, Tokyo 108-8642 (JP)
(74) Representative: Grünecker, Kinkeldey, Stockmair & Schwanhäusser Anwaltssozietät
(86) International application number: PCT/JP2000/001289
(87) International publication number: WO 2000/051634

(56) References cited:
- WO-A1-96/06627
- WO-A1-96/32964
- JP-A- 5 009 130
- JP-A- 7 173 069
- PARMENTIER HENK K ET AL: "Dietary unsaturated fatty acids affect antibody responses and growth of chickens divergently selected for humoral responses to sheep red blood cells" POULTRY SCIENCE, vol. 76, no. 8, 1997, pages 1164-1171, XP002311481 ISSN: 0032-5791
- YAMADA, HARUKI ET. AL.: 'In vivo Antiinfluenza Virus Activity of Kampo Medicine Sho-Seiryu-To Through Mucosal immune System.' METH. FIND. EXP. CLIN. PHARMACOL. vol. 20, no. 3, 1998, pages 185 - 192, XP002928712
- MARUNO, MASAO: 'Research for active principles of Pinelliae Tuber and new preparation of crude drug.' JOURNAL OF TRADITIONAL MEDICINES vol. 14, 1997, pages 81 - 88, XP002928713
- MASUI ET AL: "An antifungal compound, 9,12,13-trihydroxy-(E)-octadecenoic acid from colocasia antiquorum inoculated with ceratocystis fimbriata" vol. 28, no. 10, 1989, pages 2613-2615,

## Description

### Technical Field

The present invention relates to an adjuvant that contains a hydroxy unsaturated fatty acid as an active ingredient and to vaccine preparations containing the adjuvant as a constituent, such vaccine preparations being useful to prevent or treat diseases of human and other animals.

### Background Art

Vaccines have been used to prevent various diseases, and have provided tremendous and excellent results in the prevention of specific diseases such as smallpox. Nonetheless, vaccines also have side effects and there are many cases in which vaccines are less effective. Thus there is much room for improvement in the field of vaccines. Currently, many types of vaccines used for human or other animals are prepared by using pathogenic organisms, or parts thereof, as antigenic materials for vaccine production. Thus, there is no denying the possibility that vaccines are contaminated with constituents of pathogenic organisms or ingredients of growth medium for pathogenic organisms. These contaminants can cause adverse side effects upon vaccination. In addition,- antigenic sites associated with immunization themselves can induce side effects when inoculated in large quantity.

Attempts have been made to avoid such side effects as much as possible and to manufacture safe vaccines.Such attempts include the reduction of inoculum dose of vaccine, the use of high-purity preparations of antigen for vaccine, the alteration of vaccination routes, and the like. However, these revisions have a general problem -the immunological activity of such revised vaccines tends to be reduced. Accordingly, adjuvants have been used to prevent such a decline of immunological activity. However, in such cases, there remain some problems to be improved, such as improvement in effectiveness and safety of adjuvants.

For example, a pathogenic microorganism such as influenza virus infects via mucous membranes of the respiratory tract. To prevent such diseases at early stages of infection, vaccines capable of significantly enhancing local immunity on the mucous membrane rather than systemic immunity in the blood are preferred. In this context, it is also preferable to have an adjuvant capable of contributing to the enhancement of local immunity. At the same time, instead of injection, oral or intranasal inoculation is noteworthy as a vaccination route. The injection must be performed by medical technicians and is, therefore, problematic when it is necessary to vaccinate many people under a condition with no or only poor medical facilities. On the other hand, oral and intranasal inoculation can be performed without direct practices by medically skilled staffs, so long as vaccine preparations are available. However, in general, when vaccinated with an injectable vaccine, via alternate vaccination route, sufficient immunological stimulation is difficult to attain and, therefore, certain adjuvants suitable for alternate vaccination routes are needed.

In other words, an important challenge for the development of vaccines is to develop an excellent adjuvant that is effective and safe and that helps the enhancement of required immunity at the desired site.

Previously, aluminum compounds (aluminum sulfate, aluminum hydroxide, etc.) and phosphate compounds-(calcium phosphate, aluminum phosphate, etc.) have widely been used as adjuvants for vaccination. Currently, the gel of these compounds is almost the only adjuvant that is used for human vaccination. However, there are some problems in regard to these adjuvants, and thus the adjuvants are in need of improvement. Some illustrations are as follows: 1) Problems associated with manufacturing and handling: For example, since the quality of these adjuvants tends to vary from one production lot to another, they are not suited to large-scale manufacturing. Moreover, the handling is also inconvenient. For example, they are unsuitable for column operation. 2) A problem associated with their effect: While they excel in inducing the humoral immunity, they are not effective for inducing the cellular immunity, and thus there are limitations on the types of antigens to be used.

Studies and development of new types of adjuvants, such as saponin, are proceeding in order to overcome the drawbacks. Some illustrations are as follows (See J. C. Cox et al., Vaccine 15, 248-256, 1997):
1. Surface active substances, such as saponins, etc.
2. Bacterial toxins, such as cholera toxin, etc.
3. Constituents of microorganisms or plants, such as BCG, muramyl peptide, etc.
4. Cytokines, such as interleukins, etc.
5. Synthetic polyanion, polycation, etc.
6. Micro-carriers, etc.

The present inventors have found that certain extracts of Chinese and Japanese traditional (Kampo) medicine, consisting of several crude drugs , exhibit adjuvant activity and increase the antibody titer against influenza virus in the nasal irrigation liquid and in the serum when used as an ingredient of influenza vaccine to be inoculated intranasally (H. Yamada and T. Nagai, Methods and Findings in Experimental and Clinical Pharmacology, 20(3), 185-192, 1998). However, exactly which component(s) of the extract has the adjuvant activity remains to be clarified.

Masui et al., Phytochemistry (1989), 28(10), pp 2613-2615, discloses an unsaturated fatty acid with 18 carbon atoms that has a trihydroxy-monoene structure and its use as antifungal.

### Disclosure of the Invention

An objective of the present invention is to provide a novel method for enhancing the immunological activity of vaccine in order to produce vaccines whose immunological activity is not reduced when dosage is lowered or vaccination route is altered. More specifically, the objective is to screen for an effective and safe compound having a simpler structure among crude drug and to thereby develop a novel adjuvant. Chinese and Japanese traditional (Kampo) medicine has long been used clinically in China, Japan, and other Asian countries, and its effectiveness and safety have been already established. Thus, the medicine is excellent and suitable as the material to be utilized for the present objective.

In other words, an objective of the present invention is to provide a hydroxy unsaturated fatty acid and derivatives thereof as novel, effective, and safe vaccine adjuvants, to provide vaccines composed of these, and to contribute to the manufacture of effective and safe vaccines.

The inventors have previously revealed that a hot-water extract from a Chinese and Japanese traditional medicine "Sho-seiryu-to", which consists of 8 kinds of medicinal plants, has an adjuvant activity, and that the extract elevates the antibody titer against influenza virus in the nasal irrigation liquid as well as in the serum when orally administered in combination with intranasal inoculation of influenza vaccine (H. Yamada and T. Nagai, Methods and Findings in Experimental and Clinical Pharmacology, 20 (3), 185-192, 1998).

Thus, for the purpose of achievement of the above-mentioned objective, the inventors used hot-water extracts from the respective 8-kind medicinal plants, which are components of Sho-seiryu-to, as adjuvants to orally administer them in order to determine which component (s) exhibits the adjuvant activity to influenza vaccine upon nasal vaccination. The result showed that the hot-water extract from a medicinal plant "Pinelliae Tuber" had the highest adjuvant activity. Further, the inventors then separated and purified an active ingredient from the hot-water extract of Pinelliae Tuber and performed structural analysis thereon. The inventors found that a compound having a particular structure exhibited the strong activity of enhancing the immunity; and thus they completed the present invention. Specifically, the above-mentioned objective can be established by the preparing this inventive adjuvant and using it in a vaccine preparation. Accordingly, the present invention provides:
(1) an adjuvant comprising a hydroxy unsaturated fatty acid or a derivative thereof;
   wherein the hydroxy unsaturated fatty acid or the derivative thereof is an unsaturated fatty acid with 18 carbon atoms, or a derivative thereof, that has a trihydroxy-monoene structure;
(2) the adjuvant of (1), wherein the unsaturated fatty acid with 18 carbon atoms , or the derivative thereof, that has a trihydroxy-monoene structure is 9,12,13-trihydroxy-10E-octadecenoic acid or a derivative thereof of which structure is as follows: 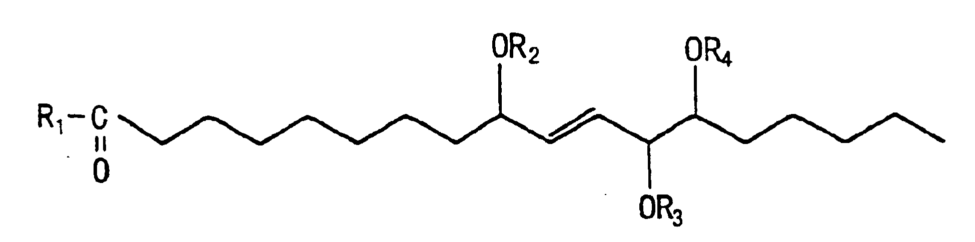 wherein R1 is a hydroxyl group, or a substituent of which structure comprises a linkage of 1 or 2 alkyl groups or aryl groups to 1 oxygen, sulfur, or nitrogen atom; and R2, R3, and 5 R4 may be identical or different and represent hydrogen, an alkyl group, or an acyl group;
(3) the adjuvant of any one of (1) to (2), wherein the hydroxy unsaturated fatty acid is a hydroxy unsaturated fatty acid or a derivative thereof prepared from a medicinal plant;
(4) a vaccine preparation comprising the adjuvant of any one of (1) to (3) as a constituent;
(5) the vaccine preparation of (4), wherein the adjuvant in the vaccine preparation is used in oral inoculation, independently of an antigen constituent;
(6) the vaccine preparation of (5), wherein an antigen constituent in the vaccine preparation is introduced through intranasal, subcutaneous, oral, or intramuscular inoculation or inoculated through other mucosae;
(7) the vaccine preparation of any one of (4) to (6), wherein the vaccine preparation comprises, as a vaccine, antigens from one or more pathogenic microorganisms selected from the group consisting of influenza virus, rotavirus, measles virus, rubella virus, mumps virus, AIDS virus, *Bordetella pertussis,* diphtheria bacillus, *Helicobacter pylori,* enterohaemorrhagic *Escherichia coli* (EHEC), *Chlamydia*, *Mycoplasma,* Malaria *Plasmodium*, coccidium, and schistosome;
(8) a medical use administering the vaccine preparation of (4), the method comprising orally administering the adjuvant in the vaccine preparation independently of the antigen constituent; and
(9) the method of (8), wherein the antigen constituent is administered intranasally, subcutaneously, orally, or intramuscularly, or through other mucosae.

The term "adjuvant" in the present invention refers to a substance capable of stimulating the immune system and thereby enhancing the immune response to an antigen.

Also, such a phrase "vaccine preparation comprising an adjuvant as a constituent" in the present invention encompasses not only the embodiment wherein the adjuvant is mixed with other constituents that can be components of a vaccine preparation, such as immunogenic constituents, but also the embodiment wherein the adjuvant is separated from other constituents that can be components of a vaccine preparation, such as immunogenic constituents. For example, even when an antigen constituent and an adjuvant are prepared separately and administered into a living body through an independent route, the two together are referred to as a vaccine preparation.

The adjuvant of the present invention is characterized by being a hydroxy unsaturated fatty acid to be used as an adjuvant belonging to a class of compounds comprising 18 carbons and having 3 hydroxyl groups and a double bond (more particularly, having a trihydroxy-monoene structure) . Such a compound is novel as a fatty acid adjuvant, in terms of containing hydroxyl groups and a double bond on the chain of fatty acid thereof. The hydroxyl groups and the double bond on the chain of fatty acid can-be positioned on any carbons except those of the carboxylic acid. Also, when each hydroxyl group is separately linked to a different carbon, the hydroxyl group can be in a R- or S-configuration, and both configurations are allowable in the present invention. Further, the existence of two modes of linkage between the double bond and the substituent results in two configurations represented by E and Z; in this case, both configurations are also allowable.

In the context of maintaining or improving adjuvant activity, preferable positions of the hydroxyl groups and the double bond are exemplified as follows: hydroxyl groups are preferably at the positions of 9,12,and 13; and position and configuration of the double bond are preferably 10 and E, respectively. Such compounds includes, for example, 9,12,13-trihydroxy-10E-octadecenoic acid. Some published reports describe this compound as having an activity of inhibiting angiotensin converting enzyme and phytoalexin activity in rice plant; however, there are no reports on the adjuvant activity in vaccination (M. Maruno, J. Traditional Medicines, 14, 81-88, 1997; T. Kato, Y. Yamaguchi, T. Uyehara, T. Yokoyama, T. Namai and S. Yamanaka, Naturwissenschaften, 70, 200, 1983; T. Kato, Y. Yamaguchi, N. Abe, T. Uyehara, T. Namai, M. Kodama and Y. Shiobara, Tetrahedron Lett., 26, 2357-2360, 1985).

The adjuvant of the present invention includes derivatives in which various substituents are linked to the hydroxyl groups of the above-mentioned fatty acid and the carbonyl group of the carboxylate moiety thereof. Such derivatives include, for example, ester derivatives, in which an acyl group, such as acetyl group, benzoyl group, pyruvate group, or succinate group, is linked to the hydroxyl group; as well as ether derivatives, in which an alkyl group, such as ethyl group or methyl group, is linked to it. Further examples of substituents linked to the carbonyl group of carboxylate include: alkyloxy groups, such as hydroxyl group, ethyloxy group; aryloxy groups, such as benzyloxy group; thioalkyl groups, such as thioethyl group, or thioaryl group, amino group, primary amine, or secondary amine, etc.

Specifically such compounds include, for example, 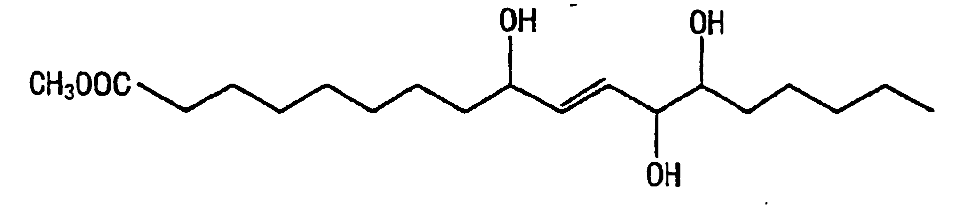 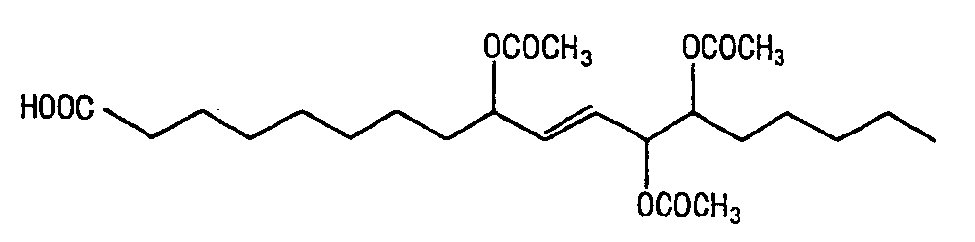 , and

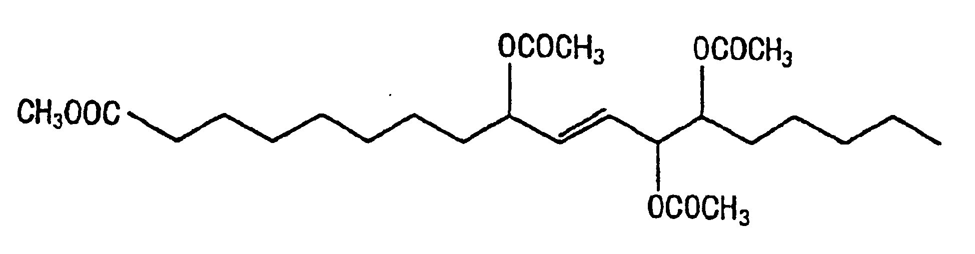

There are no reports on the strong adjuvant activity of a hydroxy unsaturated fatty acid having a trihydroxy-monoene structure in previously published literatures. The finding is novel and was first revealed by the present inventors based on their studies for long years. As described below, it is impossible to predict it, even based on descriptions in previous reports.

Known fatty acid compounds having adjuvant activity, of which structures have already been clarified, include linoleic acid and arachidonic acid (H.K. Parmentier, M.G.B. Nieuwland, M.W. Barwegen, R.P. Kwakkel and J.W. Schrama, Poultry Science, 76 (8), 1164-1171, 1997; D.S. Kelley, P.C. Taylor, G.J. Nelson, P.C. Schmidt, B.E. Mackey and D. Kyle, Lipids, 32 (4), 449-456, 1997). Firstly, although having 18 carbons, linoleic acid is different from the compound of the present invention in that it is a dienoic acid, which contains two double bonds; moreover, it is clearly distinct from the inventive compound in that it has no hydroxyl group. Arachidonic acid is a fatty acid having 20 carbons, 4 double bonds, and no hydroxyl group, and thus this compound is different from the inventive fatty acid.

So far it still remains to be clarified what mechanism underlies the strong adjuvant activity of the inventive hydroxy unsaturated fatty acid. However, the inventors have revealed that orally administered Chinese and Japanese traditional medicine "Sho-seiryu-to" exhibits the adjuvant activity of increasing the titer of anti-influenza virus IgA antibody in the nasal cavity when influenza vaccine is intranasally inoculated (T. Nagai, M. Urata and H. Yamada, Immunopharmacology and Immunotoxicology 18(2), 193-208, 1996). Further, the inventors have also found that oral administration of "Sho-seiryu-to" activates T lymphocytes in Peyer's patches, a tissue associated with induction of the mucosal immune system in the intestinal tract, as well as increases the number of cells producing influenza virus-specific IgA antibody among lymphocytes located in the nasal cavity (H. Yamada and T. Nagai, Methods and Findings in Experimental and Clinical Pharmacology 20 (3), 185-192, 1998; T. Nagai and H. Yamada, Immunopharmacology and Immunotoxicology 20(2), 267-281, 1998). There exists a common mucosal immune system in the mucosal immunity, and thus activation of any one of mucosal immune systems in the body results in the activation of other mucosal immune systems in other body areas, through distant immunity. Because the hydroxy unsaturated fatty acid that is the adjuvant of the present invention has been identified as an essential substance exhibiting the adjuvant activity contained in "Sho-seiryu-to," it is possible that the fatty acid, like "Sho-seiryu-to", activates the mucosal immune system in the intestinal tract to enhance the production of anti-influenza virus IgA antibody in the nasal cavity and thereby exhibiting the adjuvant activity.

### Production of the hydroxy unsaturated fatty acid

The fatty acids to be used in the present invention can be extracted, separated, purified, and manufactured from natural products, for example, animal tissues, medicinal plants, marine plants, and cultures of microorganisms used as raw material, by the combined use of known methods. They can also be manufactured by means of chemical synthesis. Examples of the fatty acid are as follows:

A medicinal plant containing the fatty acid, Pinelliae Tuber (rhizome of Pinellia ternata Breit. except for the cork layer thereof), is subjected to extraction with an organic solvent, such as methanol or acetone, and then the solvent is distilled off from the extract. The resulting residue is dissolved in water-containing methanol and then extracted with a low polar solvent, such as n-hexane or petroleum ether. The solvent is distilled off from the water-containing methanol layer, and then the resulting residue is fractionated, once or several times, by column chromatography using Sephadex, such as Sephadex LH-20, a porous polymer such as DIAION HP-20, and a carrier such as alumina or silica gel by using at least one elution solvent selected from the group consisting of water, methanol, ethanol, chloroform, ether, n-hexane, benzene, and ethyl acetate. The constituent of interest is monitored by thin-layer chromatography; thus, the preparation of the fatty acid is successfully achieved. Alternatively, after the extraction of Pinelliae Tuber with water or the like, it can be purified from the resulting water extract by ethanol precipitation or fractionation, using a porous polymer such as DIAION HP-20 and a silica gel column chromatography. Alternatively, in some cases, it can be purified by recrystallization, using an appropriate solvent, such as acetone, methanol, ethanol, etc.

Further, if desired, various derivatives can be prepared from the compound obtained as described above through methylation, ethylation, or benzoylation, by properly combining known chemical, biochemical, and genetic techniques.

The structure of compounds of the present invention can be analyzed by known methods, such as mass spectrometry and nuclear magnetic resonance spectrum (W. Herz and P. Kulanthaivel, Phytochemistry, 24 (1), 89-91, 1985; S. Ohnuma, T. Uehara, T. Namai, M. Kodama, Y. Shiobara, Chemistry Letters, 577-580 (1986) ; M. Hamberg, Lipids, 26, 407-415 (1991); I. Ohtani, T. Kusumi, Y. Kashman, H. Kakisawa, J. American Chemical Society, 113, 4092-4096 (1991); K, Kouda, T. Ooi, K. Kaya, T. Kusumi, Tetrahedron Letters, 37, 6347-6350 (1996); M. Kobayashi, T. Tawara, T.Tsuchida, H. Mitsuhashi, Chemical Pharmaceutical Bulletin, 38, 3169-3171 (1990)).

### Vaccine

New vaccine preparations, utilizing the inventive adjuvant, are also provided. The vaccine preparations of the present invention include vaccines in both narrow and broad senses. Specifically, the vaccines include:
i) vaccines in a narrow sense, which are effective against infectious diseases of human and other animals caused by virus, bacterium, fungus, protozoan, other microorganisms. Such vaccines are exemplified by various vaccines such as influenza vaccine, pertussis vaccine, purified pertussis-diphtheria-tetanus combined vaccine, Japanese encephalitis vaccine, hepatitis A vaccine, hepatitis B vaccine, rotavirus vaccine, measles vaccine, rubella vaccine, mumps vaccine, measles-rubella-mumps combined vaccine, measles-rubella combined vaccine, and *Haemophilus influenzae* vaccine. The vaccines also include multi-drug resistant Staphylococcus aureus (MRSA) vaccine, *Helicobacter pylori* (abbreviated as *H*. *pyroli* hereafter) vaccine, enterohaemorrhagic *Escherichia coli* (EHEC) vaccine, *Salmonella* vaccine, *Chlamydia* vaccine, *Mycoplasma* vaccine, AIDS vaccine, malaria vaccine, coccidium vaccine, and schistosome vaccine.
ii)the vaccines in a broad sense are exemplified by vaccines, which are effective in the prevention and treatment of non-infectious diseases, such as cancer vaccine, infertility vaccine, gastric ulcer vaccine, diabetic vaccine, and arteriosclerotic vaccine.

These vaccines include various vaccines that are categorized based on the types of methods to produce them. Specifically, the vaccines include attenuated live vaccines, inactivated vaccines, component vaccines, vaccine using DNA, and the like. The vaccines using DNA include vaccines containing a DNA fragment integrated in a carrier, such as plasmid, and vaccines used in combination with ribozymes or antisense oligonucleotides, and the like. These vaccines can be used for prevention and/or treatment. The vaccines also include recombinant vaccines containing, as their active ingredient, the antigen produced in cells, engineered by gene recombination techniques. These vaccines may be plain vaccines or combined vaccines. Exemplary production methods and usage forms of the vaccines are described below.

Influenza vaccine- a split vaccine containing hemagglutinin (HA), neuraminidase (NA), nuclear protein (NP), matrix protein (M), or a part of these, that is obtained by the following steps: growing the viruses in embryonated eggs or in Vero cells by using animal cell culture techniques; degrading the viruses with ether, detergent, etc.; and purifying, or that is obtained by gene recombination techniques or chemical synthesis; or a DNA vaccine for intranasal inoculation that contains DNA fragments containing genes encoding these proteins.

Pertussis vaccine- an inactivated vaccine that is obtained by the following steps: culturing *Bordetella pertussis,* treating the culture supernatant or bacteria by salting-out, ultracentrifugation to extract constituents of interest, and detoxicating with formalin; or vaccine containing mutant pertussis toxin (PT), filamentous hemagglutinin (FHA), 69 K membrane protein, or a partial peptide of these, that is prepared by gene recombination techniques or prepared as a product of an artificial mutant strain obtained by treatment with a mutagenizing agent.

Pertussis-diphtheria-tetanus combined vaccine - a triple vaccine prepared by mixing the above-described pertussis vaccine with diphtheria toxoid (DT) and tetanus toxoid (TT).

Japanese encephalitis vaccine - an inactivated vaccine that is obtained by the following steps: growing the viruses in mouse brain or in Vero cells using animal cell culture techniques; purifying the virus particles by ultracentrifugation-or with ethyl alcohol, and inactivating the virus with formalin; or a vaccine containing antigen proteins obtained by gene recombination techniques or chemical synthesis.

Hepatitis B vaccine - a plasma vaccine that is obtained by separating and purifying HBs antigen, by salting-out and ultracentrifugation, using blood collected from hepatitis B carriers as raw material; or a recombinant vaccine containing the antigen portions obtained by gene recombination techniques or chemical synthesis.

Measles vaccine - a live vaccine of an attenuated virus that is prepared by growing the virus in culture cells, such as chicken embryonic cells or in Vero cells, using cell line culture techniques; a recombinant vaccine containing a part of the virus; or a recombinant vaccine containing the protective antigen prepared by gene recombination techniques or chemical synthesis.

Rubella vaccine - a vaccine containing the viruses grown in culture cells, such as animal cells or human fetal cells or in Vero cells, using cell line culture techniques; a part of the virus; or the protective antigen prepared by gene recombination techniques or chemical synthesis.

Mumps vaccine - an attenuated live vaccine containing the viruses grown in culture cells, such as rabbit cells or in embryonated eggs; a part of the virus; or the protective antigen prepared by gene recombination techniques or chemical synthesis.

Measles-rubella combined vaccine - a dual vaccine that is obtained by mixing the above-described measles and rubella vaccines.

Measles-rubella-mumps combined vaccine - a triple vaccine that is obtained by mixing the above-described measles vaccine, rubella vaccine, and mumps vaccine.

Rotavirus vaccine - a vaccine containing the viruses grown in culture cells, such as MA104 cell; the viruses collected from patient's feces; a part of the viruses; or the protective antigen prepared by gene recombination techniques or chemical synthesis.

AIDS vaccine - a vaccine containing the viruses grown in culture cells; the viruses obtained from patients; a part of these; the protective antigen prepared by gene recombination techniques or chemical synthesis; or a DNA vaccine containing effective DNA fragments.

*H*. *pylori* vaccine - a vaccine containing, as antigens, lysate of cultured *H. pylori,* or urease, heat shock protein, toxin, and others separated from cultured *H*. *pylori;* or a vaccine for injection, oral inoculation, or intranasal inoculation that comprises these antigen proteins produced by gene recombination techniques.

### Usage pattern of adjuvant

There is no particular limitation on the usage pattern for the adjuvants of the present invention as an active ingredient in a vaccine. In other words, the adjuvant can be used with various known appropriate usage patterns. For example, the adjuvant may be part of a physically mixed preparation or a complex chemically linked with an antigen protein. In addition, the adjuvant can be incorporated together with a vaccine in a carrier such as liposome.

The adjuvants of the invention can be used concurrently together with one or more conventional adjuvants. A preferable combination of adjuvants of the present invention and conventional adjuvants can be empirically discovered under conditions to be considered, such as, the type of antigens used as immunogens, the species of animals subjected to inoculation, safety, etc. Based on the result obtained, it is possible to reduce adverse side reactions and enhance the immunoreactivity, for example, by reducing the amount of antigen or the other adjuvant.

### Method for mixing adjuvant

The inventive vaccine preparation can be prepared by mixing the above-mentioned immunogen with the inventive adjuvant at an adequate mixing ratio. The inventive vaccine preparation can be effective even when the vaccine antigen (antigen constituent) and the inventive adjuvants are separately prepared as pharmaceutical preparations and then, as shown in the Examples, the two are separately inoculated, or the two are mixed with each other at the time of inoculation. The preparation must be done under strictly sterile conditions. Each of raw materials must be completely sterile. As a matter of course, to the extent practical and possible, it is preferable that contaminated proteins that are unnecessary for vaccination, particularly those that act as pyrogens or allergens, are eliminated. Methods to achieve the treatment are known to those skilled in the art.

### Ratio of adjuvant

The volume ratio between vaccine antigen (antigen constituent) and adjuvants in vaccine preparations of the present invention can range, for example, from 1:0.0001 to 1:10,000 (weight ratio). The above range is merely a typical example. A suitable ratio is selected depending on the type of vaccine. Methods required for the selection are known to those skilled in the art.

### Properties of vaccine

The above vaccines are provided as liquid forms or powdered forms. If a powdered form is desired, the vaccines can be prepared as pharmaceutical preparations by a conventional method, including freeze-drying. Liquid forms of the pharmaceutical preparations are often suitable for the intranasal inoculation (intranasal spray, intranasal instillation, spread, etc.), oral administration, and injection. Alternatively, the intranasal inoculation can be provided as a powder spray. The inventive vaccine preparations can also be formulated with publicly known stabilizers or preservatives. Such stabilizers include about 0.1 to 0.2% gelatin or dextran, 0.5 to 1% sodium glutamate, about 5% lactose, about 2% sorbitol, etc. Known preservatives include about 0.01% thimerosal, about 0.1% β-propionolactone, and about 0.5% 2-phenoxyethanol.

### Method for inoculating vaccine formulations

The vaccine preparation of the present invention can be utilized by any conventionally known method.

When the inventive vaccine preparation is used, a mixture of a vaccine antigen (antigen constituent) and the adjuvant constituent can be used for the inoculation, or alternatively each constituent can be inoculated separately. The inoculation is preferably administered by oral or intranasal route. The effect of enhancing immunity can be achieved, even when the respective constituents are inoculated separately, for example, even when the vaccine antigen (antigen constituent) is intranasally inoculated and the adjuvant constituent is orally administered.

The dose is preferably 5 to 50 µl in intranasal inoculation or 0.05 to 0.5 ml in oral administration to mouse. The dose preferably ranges from about 0.1 to 1.0 ml in intranasal administration or about 1 to 100 ml in oral administration to human. The dose is changeable when desired. Regarding the combination with immunological antigen, for example, it has been believed that the following immunological antigens of pathogenic microorganism are advantageously inoculated intranasally or orally in terms of vaccination effect or inoculation procedure:influenza virus, rotavirus, measles virus, rubella virus, mumps virus, human immunodeficiency virus, *Bordetella pertussis,* diphtheria bacillus, *H. pylori,* enterohaemorrhagic *Escherichia coli* (EHEC), *Chlamydia, Mycoplasma,* Malaria *Plasmodium,* coccidium, and schistosome.

These vaccine antigens (antigen constituent) and adjuvants can be inoculated singly or concurrently, for example, like pertussis-diphtheria-tetanus triple vaccine or measles-rubella dual vaccine. The intranasal and oral inoculations are preferable, because mucous membranes of the respiratory tract and digestive tract can be infection routes. A suitable adjuvant, whose immunity-inducing activity is strong, is preferable in order to induce immunity in local mucous membranes, which can be primary infection routes. Further, some vaccinations, such as vaccination against Malaria *Plasmodium,* are performed in most cases in regions without sufficient medical facilities. In such occasions, it is advantageous to select a vaccination route such as intranasal or oral inoculation route, thereby allowing a person who is not a technician, such as physician or nurse, to perform the vaccination.

### Brief Description of the Drawings

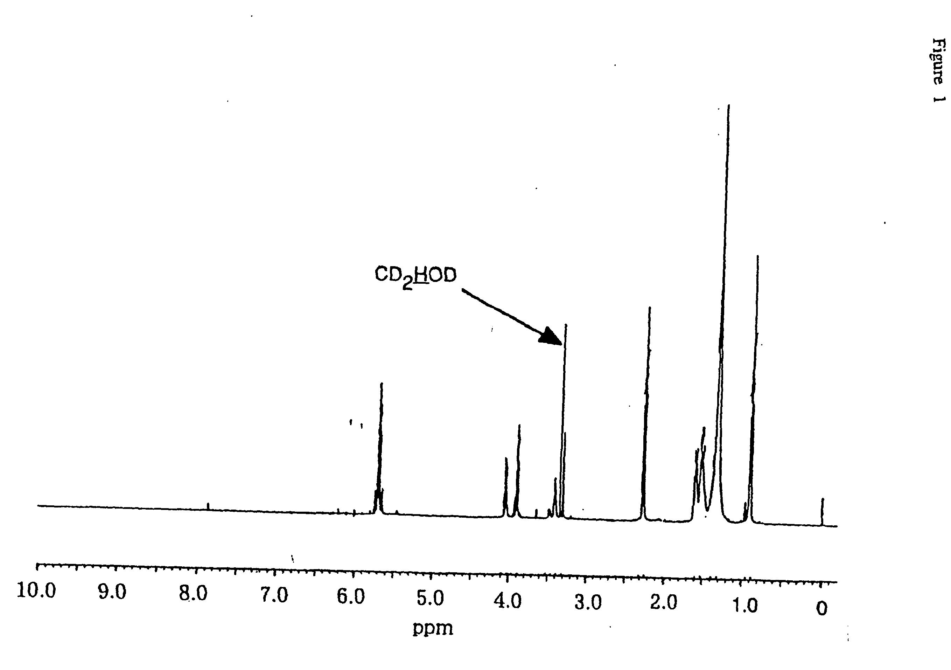

Figure 1 is a graph showing a ¹H-NMR pattern of 9,12,13-trihydroxy-10E-octadecenoic acid, which is the preferred adjuvant of the present invention. The "CD₂HOD" in this figure represents a signal from the solvent.

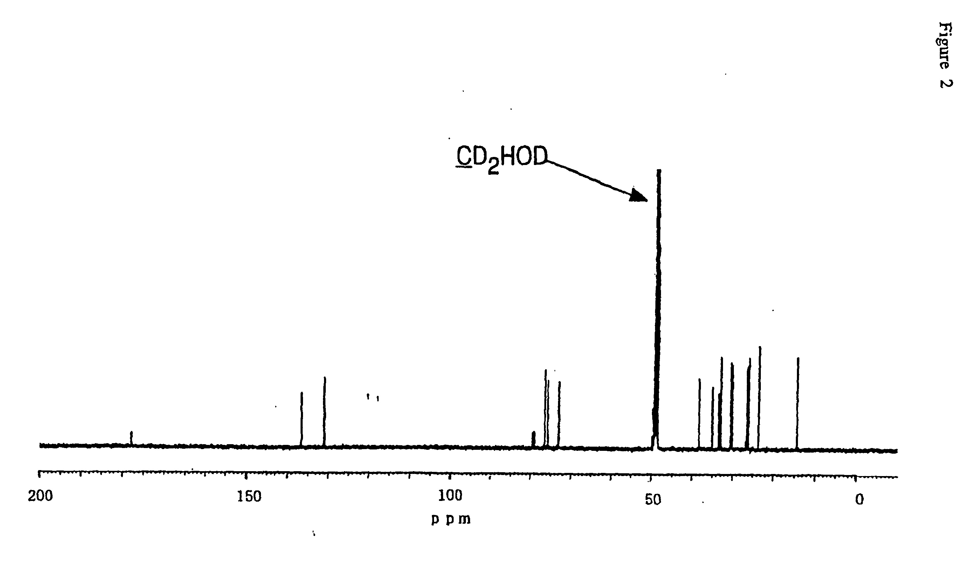

Figure 2 is a graph showing a ¹³C-NMR pattern of 9,12,13-trihydroxy-10E-octadecenoic acid, which is the preferred adjuvant of the present invention. The "CD₂HOD" in this figure represents a signal from the solvent.

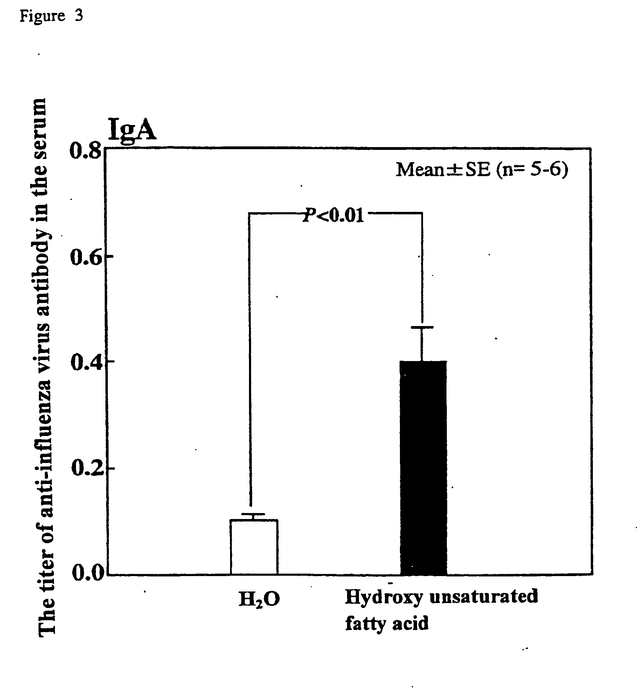

Figure 3 is a graph of primary production of antibody in the serum when an influenza vaccine is intranasally inoculated. In this figure, the ordinate indicates the antibody titer (ELISA unit) and the abscissa indicates the type of adjuvant used.

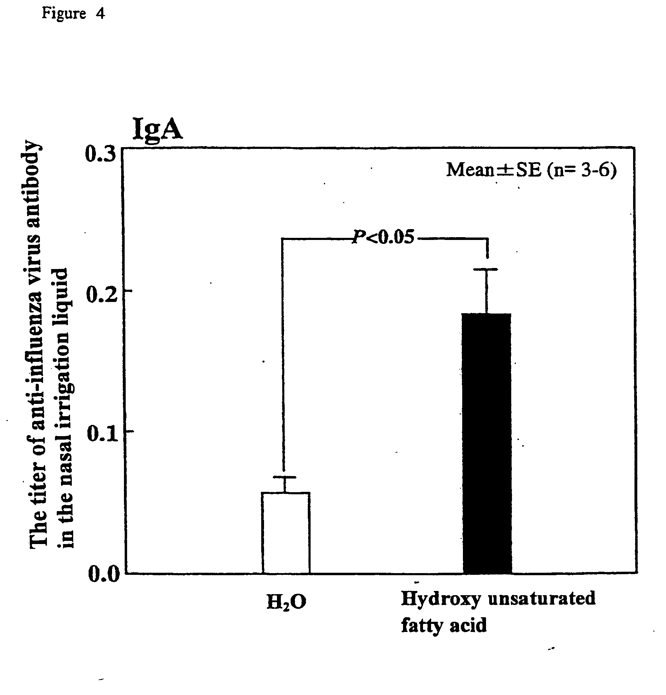

Figure 4 is a graph of secondary production of antibody in the nasal irrigation liquid resulting from the intranasal inoculation of an influenza vaccine. In this figure, the ordinate indicates the antibody titer (ELISA unit) and the abscissa indicates the type of adjuvant used.

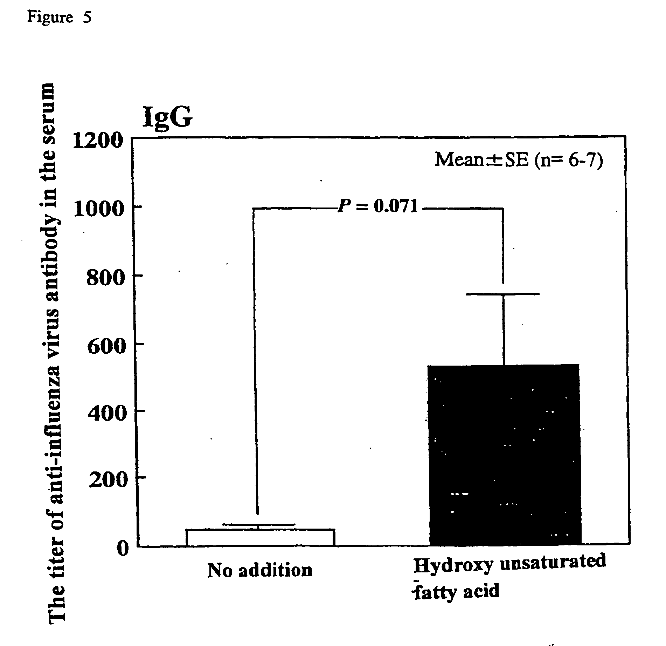

Figure 5 is a graph of secondary antibody production in the serum resulting from intranasal inoculation with an influenza vaccine and intranasal administration of an adjuvant. In this figure, the antibody titer (ELISA unit) is indicated in the ordinate axis and the type of adjuvant used is indicated in the abscissa axis.

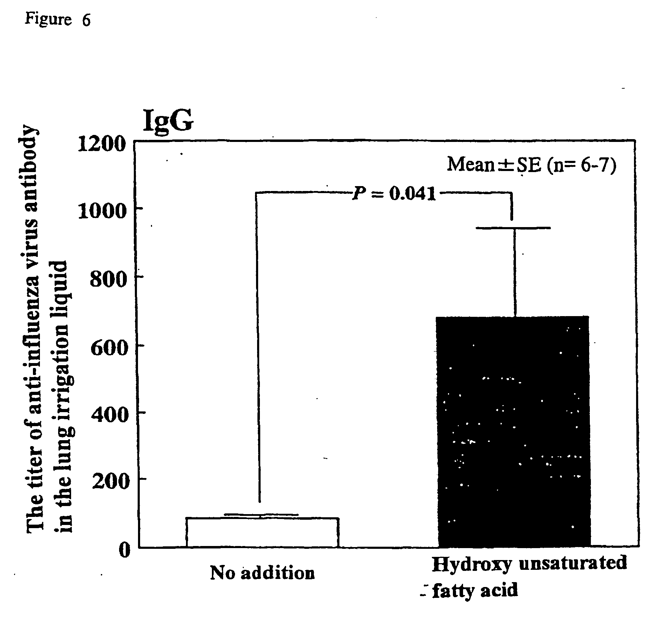

Figure 6 is a graph of secondary antibody production in the lung irrigation liquids resulting from intranasal inoculation with an influenza vaccine and intranasal administration of an adjuvant. In this figure, the antibody titer (ELISA unit) is indicated in the ordinate axis and the type of adjuvant used is indicated in the abscissa axis.

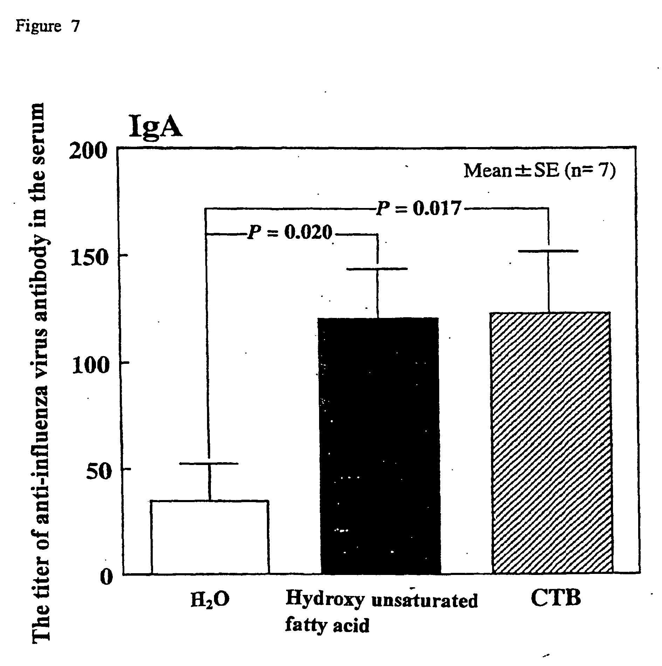

Figure 7 is a graph of secondary antibody production in the serum resulting from the subcutaneous inoculation with an influenza vaccine and the oral administration of an adjuvant. In this figure, the antibody titer (ELISA unit) is indicated in the ordinate axis and the type of adjuvant used is indicated in the abscissa axis.

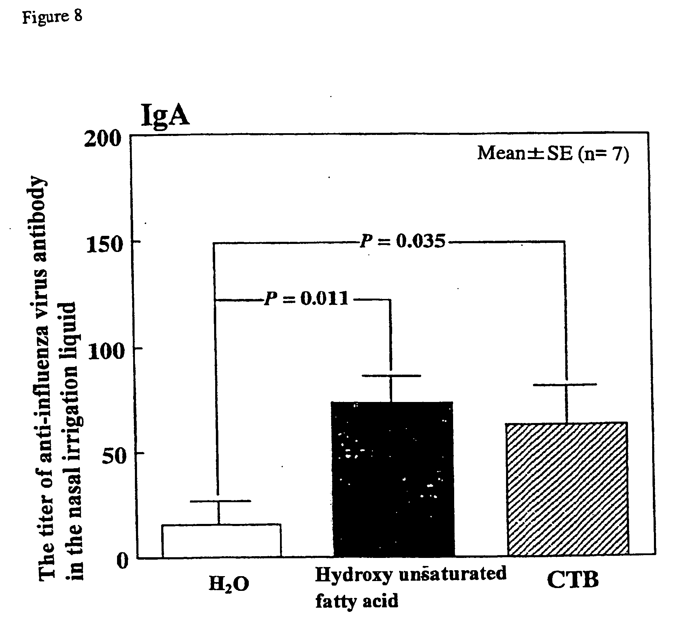

Figure 8 is a graph of secondary antibody production in the nasal irrigation liquids resulting from the subcutaneous inoculation with an influenza vaccine and the oral administration of an adjuvant. In this figure, the antibody titer (ELISA unit) is indicated in the ordinate axis and the type of adjuvant used is indicated in the abscissa axis.

### Best Mode for Carrying out the Invention

Examples of the present invention are illustrated below, but the present invention is not to be construed as being limited thereto.

### Example 1. Preparation of hydroxy unsaturated fatty acid - (1)

9,12,13-Trihydroxy-10E-octadecenoic acid was manufactured according to the method as described in Unexamined Published Japanese Patent Application (JP-A) No. Hei 3-258775 entitled "Fatty acid compound and antihypertensive agent comprising as an active ingredient the fatty acid compound." , the contents of which are incorporated by reference herein.

1 kg of Pinelliae Tuber was methanol-extracted by heating, and then the solvent was distilled off from the extract under reduced pressure to give 21.2 g of methanol-extracted material. The methanol-extract was dissolved in 100 mL of 90%(v/v) methanol-water mixed solution and then transferred into a separatory funnel. After 50 mL of n-hexane was added, the funnel was shaken gently and then allowed to stand. The lower layer was recovered and concentrated up to half of the initial volume. The resultant concentrate was subjected to hydrophobic chromatography using a DIAION HP-20 (Mitsubishi Chemical) column. The elution was performed firstly with water, then with a 50% (v/v) methanol-water mixed solution, and finally with methanol. A 530-mg fraction of the elute with methanol was subjected to column chromatography using Sephadex LH-20 (Amersham-Pharmacia Biotech), then to normal phase column chromatography using silica gel, and finally to reverse phase high performance liquid chromatography using µ-Bondapak C18 (Waters) column to give 9,12,13-trihydroxy-10E-octadecenoic acid as a colorless oily substance. The yield was 10 mg. The structure of the oily substance was determined by studying the compound and derivatives thereof with mass spectrometry, nuclear magnetic resonance spectrum (NMR), specific rotation, or others. ¹H-NMR and ¹³C-NMR patterns of the substance are shown in Figures 1 and 2, respectively.

### Example 2. Preparation of hydroxy unsaturated fatty acid - 2

This Example describes the preparation of 9,12,13-trihydroxy-10E-octadecenoic acid of the present invention from a hot-water extract of Pinelliae Tuber.

500 g of Pinelliae Tuber was decocted with 10 L water until volume of the solution was reduced up to half of the initial one, and then the resulting extract was filtered. The residue was further decocted again by the same method. Both extracts were combined together and subjected to freeze-drying treatment to yield a hot-water extract (yield: 19.8%). The hot-water extract was refluxed with 2.5 L of methanol to give a methanol-soluble fraction and an insoluble fraction. The methanol-insoluble fraction was subjected to the same treatment further twice. After the methanol-insoluble fraction was again dissolved in water, 4-times as much volume of ethanol was added thereto and the resulting mixture was stirred overnight. The precipitate and supernatant were separated from each other. Further, the precipitate was dialyzed against distilled water by using a cellulose membrane with molecular-weight exclusion limit of 10,000, and then the inner dialysate was subjected to freeze-drying treatment to give an undialyzable fraction (yield: 0.6%). The undialyzable fraction was dissolved in water and stirred together with DIAION HP-20. Then, the unabsorbed fraction was removed by washing the DIAION HP-20 with water. After the adsorbed substances were eluted by further washing with DIAION HP-20 with a 20%(v/v) and then with a 80%(v/v) methanol-water mixed solution, the adsorbed fraction was eluted with methanol to give a methanol-elution fraction (yield: 0.06%). The methanol-elution fraction was repeatedly fractionated by silica gel column chromatography to give the inventive 9,12,13-trihydroxy-10E-octadecenoic acid. The yield was 0.35 mg.

In addition, a methanol-soluble fraction (45.4 g) obtained from a hot-water extract of Pinelliae Tuber by methanol-refluxing was dissolved in 200 mL of methanol-water mixed solution (9:1) then extracted with an equal volume of n-hexane while being shaken to obtain the lower layer. The solvent was distilled off from the lower layer under reduced pressure, and the resulting material was stirred with DIAION HP-20 in an 80% methanol-water mixed solution. The unabsorbed fraction was removed by washing DIAION HP-20 with the same solvent. Further, the adsorbed fraction was obtained by eluting it from DIAION HP-20 with methanol. The adsorbed fraction was fractioned several times by silica gel column chromatography to give the inventive 9,12,13-trihydroxy-10E-octadecenoic acid (1.2 mg).

It was verified by known biological methods that the hydroxy unsaturated fatty acid was active as the adjuvant. The Examples are shown below to confirm that the hydroxy unsaturated fatty acid has the activity of enhancing the production of antibodies against a variety of vaccines and is active as the adjuvant.

### Example 3. The enhancing effect on antibody production in the primary immunization with influenza HA vaccine inoculated into the nasal cavity

HA vaccine (protein concentration was 1 mg/mL) was prepared from purified influenza virus (A/PR/8/34) by defatting with ether treatment. An aqueous solution of 9,12,13-trihydroxy-10E-octadecenoic acid purified by the method as described in Example 1 was prepared to give a hydroxy unsaturated fatty acid solution. The purity of 9,12,13-trihydroxy-10E-octadecenoic acid used here was about 95% or higher. The aqueous solution of hydroxy unsaturated fatty acid was given to BALB/c mice (7-week old female) at a dose of 50 µg/kg per mouse body weight for 5 days by forced intragastric administration with an oral sonde. 3 days after the initiation of oral administration of the hydroxy unsaturated fatty acid, the mice were anesthetized with sodium amobarbital and 10 µL of the vaccine was dropped into the right nasal cavity with a micro-pipette. After 2 weeks, blood was collected from the venous plexus of eyegrounds of the mice to prepare the sera. The titers of anti-influenza virus IgA antibody in the sera were determined by enzyme-linked immunosorbent assay (ELISA). The serum was loaded onto a column using Protein G Sepharose 4FF (Amersham-Pharmacia Biotech) equilibrated with 20 mM sodium phosphate buffer (pH 7.0), and then the unabsorbed fraction was obtained by washing the column with 20 mM sodium phosphate buffer (pH 7.0).

Prior to the assay for the anti-influenza virus IgA antibody, each well of the 96-well EIA plate was first coated with 100 µl of HA vaccine (5 µg/ml) suspended in a coating buffered (10 mM sodium carbonate bicarbonate buffer pH 9.6). After standing at room temperature for 2 hours, the plate was washed with phosphate buffered saline (PBS)- 0.05% Tween-20. Subsequently, each well was coated with 300 µL of a blocking solution (PBS containing 1% bovine serum albumin (BSA) and 0.1% NaN₃) to avoid non-specific reactions. After standing at 4°C overnight, the plate was washed with PBS-Tween-20. A 100 µL aliquot of test sample diluted with the blocking solution was added to each well. An unabsorbed fraction of serum on the column of Protein G Sepharose was used as a sample for the quantification of the anti-influenza virus IgA antibody. After standing at room temperature for 2 hours, the plate was washed with PBS-Tween-20. Subsequently, a 100 µL aliquot of alkaline phosphatase-conjugated goat anti-mouse IgA α-chain antibody (Zymed Laboratories) diluted with a blocking solution was added to each well. After standing at room temperature overnight, the plate was washed with PBS-Tween-20. Finally, *p*-nitrophenyl phosphate (1 mg/mL; Wako Pure Chemical Industries) dissolved in 10% diethanolamine buffer (pH 9.8) was added to each well to perform color development. After standing at 37°C for 20 to 30 minutes, the developed color (O.D. at 405 nm) was assayed in a micro-plate reader.

Figure 3 shows the influence of 9,12,13-trihydroxy-10E-octadecenoic acid on the titer of anti-influenza virus antibody in the serum. Only a low level of antibody was detected when the vaccine was given by intranasal inoculation and an aqueous solvent without the adjuvant was orally administered. However, the oral administration of 9,12,13-trihydroxy-10E-octadecenoic acid resulted in a highly elevated titer of anti-influenza virus IgA antibody in the serum. These results described above show that the oral administration of 9,12,13-trihydroxy-10E-octadecenoic acid enhances the production of antibody against intranasally inoculated influenza HA vaccine in the blood.

### Example 4. The enhancing effect on antibody production in the secondary immunization with influenza HA vaccine

Aqueous solutions of influenza HA vaccine and 9,12,13-trihydroxy-10E-octadecenoic acid were prepared according to the same methods as described in Example 3. The sample solution was given to 7-week old female BALB/c mice at a dose of 50 µg/kg per mouse body weight over 1-5 days by forced intragastric administration with an oral sonde. On the day of oral administration, or 3 days after the administration of 9,12,13-trihydroxy-10E-octadecenoic acid, the mice were anesthetized by intraperitoneally administering sodium amobarbital and 10 µL of vaccine (1 to 5 µg/mouse) was administered to the mice by intranasal inoculation. After being bred for about 3 weeks, the mice were further subjected to secondary intranasal inoculation of the vaccine alone or, alternatively, subjected to both secondary inoculation of the vaccine and oral administration of the hydroxy fatty acid adjuvant. After the mice were bred for an additional 2 weeks, nasal irrigation liquids were prepared. The nasal irrigation liquids were collected from the mice by perfusing the right and left nasal cavities twice with 2 ml of PBS containing 0.1% BSA. The titers of anti-influenza virus IgA in the nasal irrigation liquids were determined by ELISA.

Figure 4 shows the influence of 9,12,13-trihydroxy-10E-octadecenoic acid on the production of anti-influenza virus IgA antibody in the nasal irrigation liquids in the secondary response. Only a low level of anti-influenza virus IgA antibody was detected when the vaccine was intranasally inoculated and no adjuvant was administered. On the other hand, the titer of anti-influenza virus IgA antibody was highly elevated in the nasal irrigation liquids in the groups subjected to the oral administration of 9,12,13-trihydroxy-lOE-octadecenoic acid.

Subsequently, detection was carried out for the presence of antibodies (IgG and IgA) specific to the adjuvant and IgE. A linked complex between the hydroxy unsaturated-fatty acid and bovine serum albumin as a carrier protein was prepared. Each well of the 96-well EIA plate was first coated with a 100 µl aliquot of solution containing the complex (1 µg/ml). Subsequently, each well of the plate was coated with 300 µl aliquot of a blocking solution (PBS containing 5% skimmed milk) for 1 hour to avoid non-specific reactions. Then, 100 µl samples (nasal irrigation liquid) diluted to various concentrations were added into the respective wells for antigen-antibody reaction and the reaction was continued for 1 hour. The plate was then washed 3 times with PBS-0.05% Tween-20, and then 100 µl of peroxidase-conjugated anti-mouse IgG, IgA or IgE antibody (1:1000) as a secondary antibody was added thereto and the reaction was continued for one hour. After the plate was washed 3 times with PBS-Tween-20, 100 µl of a substrate solution (0.1 M citrate buffer (pH 4) containing 0.003% hydrogen peroxide and ABTS of 0.3 mg/ml) was added thereto. The plate was incubated for 15 minutes for color development. The color (O.D. at 405 nm) was assayed in a micro-plate reader. The result showed that no differences in the absorbance of nasal irrigation liquid were recognized between the group of mice to which the hydroxy unsaturated fatty acid had been administered intranasally and groups of control mice without administration. According to this result, neither antibody (IgG, IgA) specific to the adjuvant nor IgE was detectable.

As described above, the result that the titer of anti-influenza virus IgA antibody was elevated by the presence of 9,12,13-trihydroxy-lOE-octadecenoic acid shows that 9,12,13-trihydroxy-10E-octadecenoic acid orally administered at the time of primary inoculation of the vaccine has the strong effect of inducing the production of antibody in the secondary inoculation of vaccine in the respiratory tract. In other words, this means that 9,12,13-trihydroxy-10E-octadecenoic acid strongly induces the memory effect on the HA vaccine. Since the hydroxy fatty acid used is a low-molecular-weight compound, as can be predicted, the result suggests that the compound has only low antigenicity and thus hardly induces side effects.

### Example 5. Toxicity of the hydroxy unsaturated fatty acid

Acute toxicity was studied by administering to mice the hydroxy unsaturated fatty acid (1) (9,12,13-trihydroxy-10E-octadecenoic acid) prepared in Example 1 and methyl ester derivative (2) (methyl 9,12,13-trihydroxy-10E-octadecenoate), triacetyl derivative (3)(9,12,13-triacetoxy-10E-octadecenoic acid) and triacetyl methyl ester derivative (4) (methyl 9,12,13-triacetoxy-lOE-octadecenoate) prepared from the fatty acid. Structural formulae for the respective compounds (1) to (4) are shown below:

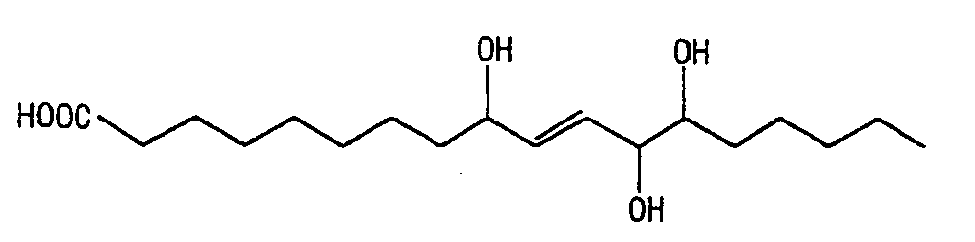

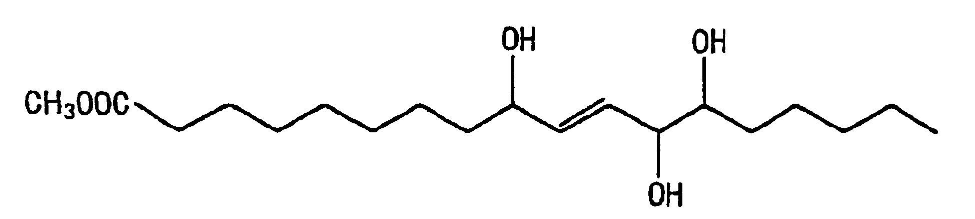

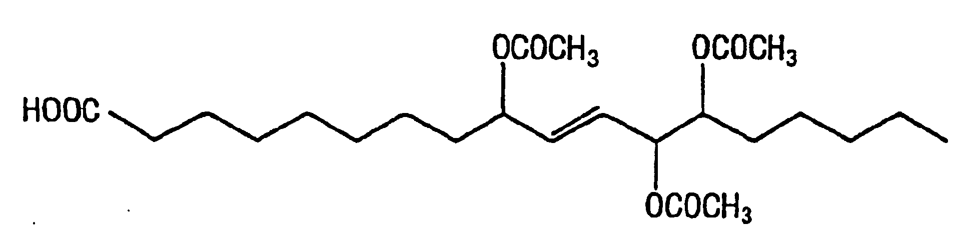

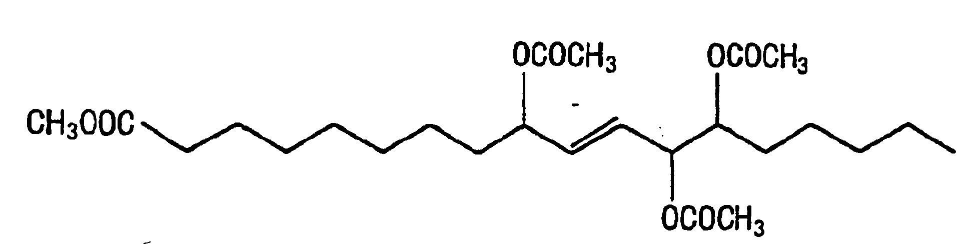

For the preparation of the compound (2) (methyl 9,12,13-trihydroxy-lOE-octadecenoate), the compound (1) (9,12,13-trihydroxy-10E-octadecenoic acid) was dissolved in ether. Subsequently excess diazomethane ether solution was added to the solution and the mixture was incubated at room temperature for several minutes. The solvent was distilled off from the reaction solution to give the compound (2).

For the compound (3) (9,12,13-triacetoxy-10E-octadecenoic acid), the compound (1) (9,12,13-trihydroxy-10E-octadecenoic acid) was refluxed in the presence of sodium acetate in acetic anhydride for about 1 hour, and then the reaction product was subjected to two-phase extraction with chloroform and water. The compound (3) was obtained from the chloroform layer.

After synthesized from the compound (1), the compound (2) was converted to compound (4) (methyl 9,12,13-triacetoxy-10E-octadecenoate) by the same method for synthesizing the compound (3).

The compounds (1) to (4) (the purities were 95% or higher) showed no sign of toxicity when intraperitoneally administered at a dose of 30 mg/kg or orally administered at a dose of 100 mg/kg.

### Example 6. Pertussis-diphtheria-tetanus combined vaccine (intranasal)-hydroxy unsaturated fatty acid (oral) preparation

The preparation was prepared to contain pertussis-diphtheria-tetanus combined vaccine of which amount corresponded to 50 µg of protein nitrogen in 20 µL. 9,12,13-Trihydroxy-10E-octadecenoic acid dissolved in PBS and sterilized by filter was prepared at a concentration of 10 µg in 0.5 mL. A preservative (0.005% thimerosal) was added to the solutions. The resulting mixtures were dispensed into containers, which were used as pertussis-diphtheria-tetanus combined vaccine (intranasal)-hydroxy unsaturated fatty acid (oral) inoculum preparations. Such preparations were stored at a temperature of not more than 10°C in a cool and dark place.

The pertussis-diphtheria-tetanus combined vaccine as prepared above was intranasally inoculated into mice, and 9,12,13-trihydroxy-10E-octadecenoic acid was orally administered at or around the time of inoculation. After 4 weeks, the same amount of vaccine was further inoculated, and then the antibody production was tested. The test result showed that the level of anti-pertussis toxin (PT)-IgG antibody was 156 ELISA units; the level of anti-diphtheria toxoid (DT)-IgG antibody was 11 ELISA units; and the level of anti-tetanus toxoid (TT)-IgG antibody was 13 ELISA units in the blood in control mice that had been inoculated with only pertussis-diphtheria-tetanus combined vaccine, but the level of anti-PT-IgG antibody was 442 ELISA units; the level of anti-DT-IgG antibody was 70 ELISA units; and the level of anti-TT-IgG antibody was 75 ELISA units in the blood in the combined use of orally administered 9,12,13-trihydroxy-lOE-octadecenoic acid. Further, while the level of anti-PT-IgA antibody was 6 ELISA units; the level of anti-PT-IgA antibody was 3 ELISA units; and the level of anti-TT-IgA antibody was 4 ELISA units in nasal irrigation liquid in control mice that had been inoculated only pertussis-diphtheria-tetanus combined vaccine, the level of anti-PT-IgA antibody was 14 ELISA units; the level of anti-PT-IgA antibody was 11 ELISA units; and the level of anti-TT-IgA antibody was 11 ELISA units in nasal irrigation liquid in the vaccination combined with the administration of 9,12,13-trihydroxy-lOE-octadecenoic acid.

### Example 7. Measles-rubella vaccine (intranasal)-hydroxy unsaturated fatty acid (oral) preparation

A measles-rubella vaccine preparation was prepared to contain virus particles of each vaccine of which amount corresponded to 7 µg in 20 µL of the preparation. 9,12, 13-Trihydroxy-10E-octadecenoic acid dissolved in PBS and sterilized by filter was prepared at a concentration of 2.5 µg in 0.5 mL. A stabilizer (0.2% porcine gelatin, 0.1% sodium glutamate, 5% lactose) was added to these preparations. The resulting mixtures were dispensed into containers, which were used as measles-rubella vaccine combined vaccine-hydroxy unsaturated fatty acid nasal drop or oral preparations. Such preparations were stored at a temperature of not more than 10°C in a cool and dark place.

The measles-rubella vaccine as prepared above was administered into mice twice at 3-week intervals, and then 9,12,13-trihydroxy-10E-octadecenoic acid was orally administered only at or around the time of the first inoculation. Then, the antibody production in the blood was evaluated. The test result showed that the ELISA titer of antibody produced was 0.14 for measles or 0.09 for rubella when the vaccine alone had been inoculated, but the titer was 0.30 for measles or 0.29 for rubella in the combined use of 9,12,13-trihydroxy-10E-octadecenoic acid with the vaccine.

### Example 8. Preparation of rotavirus vaccine-hydroxy unsaturated fatty acid ester preparation (oral preparation, nasal drop)

A rotavirus vaccine preparation was prepared to contain virus particles of which amount corresponded to 3.3 µg in 20 µL. The methyl ester derivative (2) (methyl 9,12,13-trihydroxy-10E-octadecenoate) as used in Example 5 was dissolved in PBS and prepared at a concentration of 10 µg in 0.5 mL. The resulting preparation was sterilized by filter and dispensed into containers, which were used as rotavirus vaccine-hydroxy unsaturated fatty acid oral preparations or nasal drops. Such preparations were stored at a temperature of not more than 10°C in a cool and dark place.

The rotavirus vaccine as prepared above was administered into mice twice at 3-week intervals, and then the methyl ester derivative was orally administered only at or around the time of the first inoculation. Then, the antibody production in the blood was evaluated. The test result showed that the ELISA titer of antibody produced was 0.089 in the inoculation of nasal vaccine drop when the vaccine alone was inoculated, but the titer was 0.38 in the combined use of the methyl ester derivative with the vaccine, and that the titer was 0.018 in the control group of mice without adjuvant inoculation when the vaccine had been inoculated orally, but the titer was 0.27 in the group to which the vaccine together with the methyl ester derivative had been inoculated.

### Example 9. Preparation of mycoplasma vaccine-hydroxy unsaturated fatty acid preparation (nasal drop, oral preparation)

A mycoplasma vaccine was prepared to contain mycoplasma organisms of which amount corresponded to 2.0 × 10¹⁰ CFU (colony forming unit) in 20 µL. 9,12,13-Trihydroxy-10E-octadecenoic acid dissolved in PBS and sterilized by filter was prepared at a concentration of 10 µg in 0.5 mL. These were dispensed into containers, which were used as mycoplasma vaccine-hydroxy unsaturated fatty acids preparation nasal drops or oral preparations. Such preparations were stored at a temperature of not more than 10°C in a cool and dark place.

The mycoplasma vaccine as prepared above was intranasally administered into mice 3 times at 2-week intervals, and then 9,12,13-trihydroxy-10E-octadecenoic acid was orally administered only at or around the time of the first inoculation. Then, observation was carried out for the lesions associated with Mycoplasma infection. The test result showed that the lesions were recognized in all of 10 control mice to which the vaccine alone had been administered, but the lesions were found in only 3 of 10 mice to which 9,12,13-trihydroxy-10E-octadecenoic acid had been given together with the vaccine. While the average number of lesions was 302 in the case of the vaccine alone, the number was 178 in the combined use of 9,12,13-trihydroxy-10E-octadecenoic-acid with the vaccine.

### Example 10. The enhancing effect on antibody production by the intranasal administration of the fatty acid in the secondary immunization with intranasally inoculated influenza HA vaccine

An influenza HA vaccine (the protein amount was 0.1 mg/mL) was prepared by the same method as described in Example 3. An equal volume of PBS solution of sample (0.1 mg/mL) was mixed with the vaccine to prepare an inoculum. 7-Week old female BALB/c mice were anesthetized by intraperitoneally administering sodium amobarbital. Then, a 10 µL aliquot of the inoculum was administered in each of the right and left nasal cavities. After being bred for 3 weeks, the mice were further subjected to secondary intranasal inoculation of a mixture of the vaccine and the sample. After the mice were bred for an additional 16 days, sera and lung irrigation liquids were prepared from them. After bloodletting, the lung irrigation liquids were collected from the mice by injecting 2 mL PBS containing 0.1% BSA into the mouse trachea and perfusing the lung twice. The titers of anti-influenza virus IgG antibody in the serum and lung irrigation liquids were determined by ELISA.

Prior to the assay for anti-influenza virus IgG antibody, each well of the 96-well EIA plate was first coated with 100 µL of anti-mouse IgG monoclonal antibody (mAb) (Pharmingen) diluted with a coating buffer (10 mM sodium carbonate bicarbonate buffer (pH 9.6) containing 10 µg/mL BSA). The plate was allowed to stand at 37°C for 3 hours, and then the solution in each well was discarded. Subsequently, each well was coated with 300 µL of a blocking solution (PBS containing 1% skimmed milk and 0.1% NaN₃) to avoid non-specific binding. After allowed to stand at 37°C for 1 hour, the plate was washed with PBS-Tween-20. A 100 µL aliquot of test sample diluted with the blocking solution was added to each well. After allowed to stand at room temperature overnight, the plate was washed with PBS-Tween-20. Subsequently, a 100 µL aliquot of biotin-labeled HA vaccine (1 µg/mL) diluted with the blocking solution was added to each well. After allowed to stand while being shaken at room temperature for an hour, the plate was washed with PBS-Tween-20. Subsequently, 100 µL of streptavidin-β-galactosidase diluted with the blocking solution was added to each well. After allowed to stand while being shaken at room temperature for an hour, the plate was washed with PBS-Tween-20. Further, 100 µL of 0.1 mM 4-methylumbelliferyl-β-galactoside (Sigma) dissolved in 10 mM sodium phosphate buffer (pH7.0) containing 0.1 M NaCl, 1 mM MgCl₂, 0.1% BSA and 0.1% NaN₃ was added to each well and then allowed to stand at 37°C for 2 hours. Finally, 100µL of 0.1 M glycine-NaOH buffer (pH 10.3) was added to each well and the reaction was monitored in a fluorescence plate reader (FLOW LABORATORIES) (Ex. 355 nm, Em. 460 nm).

Figure 5 shows the effect of 9,12,13-trihydroxy-10E-octadecenoic acid on the production of anti-influenza virus IgG antibody in the serum in the secondary response. 9,12,13-Trihydroxy-10E-octadecenoic acid used at a dose of 1 µg per mouse increased the titer of anti-influenza virus IgG antibody in the serum as compared with that when the HA vaccine was singly used.

Figure 6 shows the effect of 9,12,13-trihydroxy-10E-octadecenoic acid on the production of anti-influenza virus IgG antibody in the lung irrigation liquid in the secondary response. When the vaccine alone was intranasally inoculated, only a low level of anti-influenza virus IgG antibody was detected. On the other hand, the titer of anti-influenza virus IgG antibody was significantly elevated in the lung irrigation liquid in the group of mice to which 9,12,13-trihydroxy-10E-octadecenoic acid and vaccine had been inoculated at a dose of 1 µg per mouse.

These results show that 9,12,13-trihydroxy-10E-octadecenoic acid used as an adjuvant has the effect of inducing the antibody production in the serum as well as in the lung even when administered intranasally.

### Example 11. The enhancing effect on antibody production by oral administration of the fatty acid in the secondary immunization with subcutaneously inoculated influenza HA vaccine.

An influenza HA vaccine (the protein amount was 10 µg/mL) and an aqueous solution of sample were prepared by the same methods as described in Example 3. The sample solution was orally given to 7-week old female BALB/c mice at a dose of 1 µg per mouse by forced intragastric administration with an oral sonde, and 0.1 mL of the vaccine was subcutaneously inoculated in an abdominal area. After the mice were bred for 2 weeks, the sample was orally administered in the same manner and the secondary subcutaneous inoculation of the vaccine was also conducted. After the mice were bred for an additional 10 days, sera and nasal irrigation liquids were prepared from them. The titers of anti-influenza virus IgA antibody in the sera and nasal irrigation liquids were determined by ELISA. ELISA used for the quantification of anti-influenza virus IgA antibody was conducted in the same manner as in Example 10 except that an anti-mouse IgA mAb (Pharmingen) was used as a capture antibody.

Figure 7 shows the effect of 9,12,13-trihydroxy-10E-octadecenoic acid on the production of anti-influenza virus IgA antibody in the serum in the secondary response. 9,12,13-Trihydroxy-10E-octadecenoic acid used at a dose of 1 µg per mouse significantly increased the titer of anti-influenza virus IgA antibody in the serum as compared with that when the HA vaccine was singly used. In addition, the adjuvant activity of 9,12,13-trihydroxy-10E-octadecenoic acid was comparable to that of the same dose of CTB used as a positive control.

Figure 8 shows the effect of 9,12,13-trihydroxy-10E-octadecenoic acid on the production of anti-influenza virus IgA antibody in the nasal irrigation liquid in the secondary response. When the vaccine alone was subcutaneously inoculated without administered adjuvant, only a low level of anti-influenza virus IgA antibody was detected. On the other hand, the titer of anti-influenza virus IgA antibody was significantly elevated in the nasal irrigation liquid in the group of mice to which 9,12,13-trihydroxy-lOE-octadecenoic acid had been administered orally at a dose of 1 µg per mouse. In addition, the adjuvant activity of 9,12,13-trihydroxy-10E-octadecenoic acid was comparable to that of the same dose of CTB used as a positive control.

These results indicate that 9,12,13-trihydroxy-10E-octadecenoic acid used as an adjuvant, when orally administered, has the effect of inducing the production of antibody to subcutaneously inoculated vaccine (currently used vaccine) in the serum as well as in the nasal cavity.

### Industrial Applicability

The Examples shown above clearly indicate the following as the present inventive effect.
1. Oral administration of the inventive adjuvant, comprising a hydroxy unsaturated fatty acid, can enhance the production of antibody against the intranasally or subcutaneously inoculated influenza HA vaccine, etc.
2. When the inventive adjuvant is orally administered, and a vaccine antigen is inoculated through intranasal or subcutaneous route, not only the antibody production in the blood but also local antibody production (in the nasal cavity) is enhanced. In other words, the inventive adjuvant can reduce the inoculum dose of vaccine antigen to reduce the side effects.
3. Because both toxicity and antigenicity of the hydroxy unsaturated fatty acid are sufficiently low, vaccines to be used in combination with the adjuvant of the present invention are highly safe.

As discussed above, vaccine preparations containing as a constituent the adjuvant in accordance with the present invention can be expected to be effective drugs to prevent or to treat virus infection and bacterial infection by vaccination.

## Claims

1. An unsaturated fatty acid with 18 carbon atoms, or a derivative thereof, that has a trihydroxy-monoene structure, for use as an adjuvant.

2. The unsaturated fatty acid of claim 1, wherein the unsaturated fatty acid with 18 carbon atoms or the derivative thereof that has a trihydroxy-monoene structure is 9,12,13-trihydroxy-10E-octadecenoic acid, or a derivative thereof, of which structure is as follows: 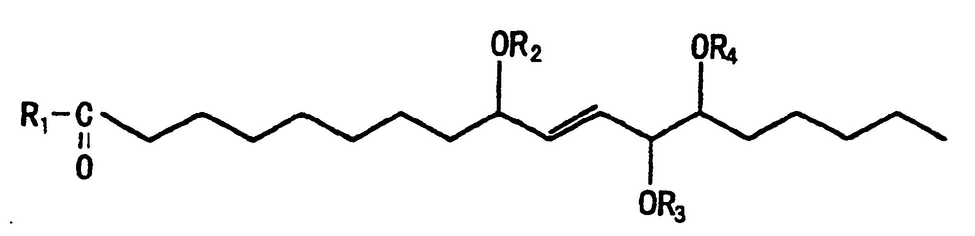 wherein R1 is selected from the group consisting of a hydroxyl group and a substituent comprising a linkage of 1 or 2 alkyl groups or aryl groups to 1 oxygen, sulfur, or nitrogen atom; and R2, R3, and R4 are independently selected from the group consisting of hydrogen, alkyl group, and acyl group and may each be identical or different.

3. The unsaturated fatty acid of any one of claims 1 or 2, wherein the unsaturated fatty acid is a hydroxy unsaturated fatty acid or a derivative thereof prepared from a medicinal plant.

4. A vaccine preparation comprising an antigen constituent and the unsaturated fatty acid of any one of claims 1 to 3 for use as an adjuvant, as a constituent.

5. The vaccine preparation of claim 4, wherein the adjuvant in the vaccine preparation is used in an oral inoculation independently of the antigen constituent.

6. The vaccine preparation of claim 5 , wherein the antigen constituent in the vaccine preparation is used in an intranasal, subcutaneous, oral, or intramuscular inoculation or is inoculated through other mucosae.

7. The vaccine preparation of any one of claims 4 to 6, wherein the antigens is derived from one or more pathogenic microorganisms selected from the group consisting of influenza virus, rotavirus, measles virus, rubella virus, mumps virus, AIDS virus, *Bordetella pertussis*, diphtheria bacillus, *Helicobacter pylori,* enterohaemorrhagic *Escherichia coli* (EHEC), *Chlamydia*, *Mycoplasma,* Malaria *Plasmodium,* coccidium, and schistosome.

8. Use of the vaccine preparation of claim 4 for preparing a medicament for the treatment of diseases, comprising orally administering the adjuvant in the vaccine preparation independently of the antigen constituent.

9. The use of claim 8, wherein the antigen constituent is inoculated intranasally, subcutaneously, orally, or intramuscularly, or through other mucosae.

## Patentansprüche

1. Ungesättigte Fettsäure mit 18 Kohlenstoffatomen oder ein Derivat derselben zur Verwendung als ein Hilfsmittel, wobei die ungesättigte Fettsäure oder das Derivat derselben eine Trihydroxy-monoenstruktur aufweist.

2. Die ungesättigte Fettsäure nach Anspruch 1, worin die ungesättigte Fettsäure mit 18 Kohlenstoffatomen oder das Derivat derselben mit einer Trihydroxy-Monoenstruktur 9,12,13-Trihydroxy-1 OE-octadecensäure oder ein Derivat derselben ist, mit der folgenden Struktur: 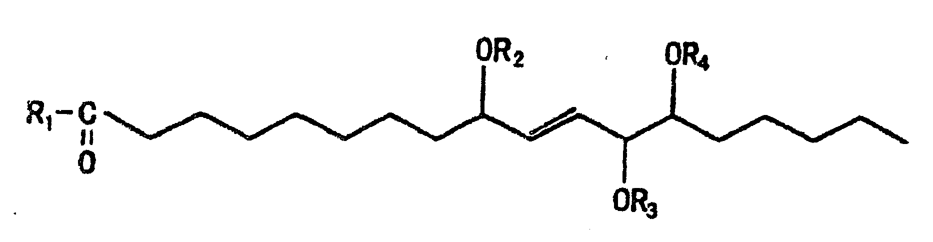 worin R1 ausgewählt ist aus der Gruppe, bestehend aus einer Hydroxylgruppe und einem Substituenten umfassend eine Bindung von einer oder zwei Alkylgruppen oder Arylgruppen an ein Sauerstoff-, Schwefel- oder Stickstoffatom; und R2, R3 und R4 unabhängig voneinander ausgewählt sind aus der Gruppe, bestehend aus Wasserstoff, einer Alkylgruppe und einer Acylgruppe, und identisch oder verschieden sein können.

3. Die ungesättigte Fettsäure nach einem der Ansprüche 1 oder 2, worin die ungesättigte Fettsäure eine Hydroxy-ungesättigte Fettsäure oder ein Derivat derselben, hergestellt aus einer medizinischen Pflanze, ist.

4. Eine Impfstoffzubereitung, umfassend einen Antigenbestandteil und eine ungesättigte Fettsäure nach einem der Ansprüche 1 bis 3 zur Verwendung als ein Hilfsmittel, als einen Bestandteil.

5. Die Impfstoffzubereitung nach Anspruch 4, worin das Hilfsmittel in der Impfstoffzubereitung unabhängig von dem Antigenbestandteil in einer oralen Impfung verwendet wird.

6. Die Impfstoffzubereitung nach Anspruch 5, worin der Antigenbestandteil in der Impfstoffzubereitung in einer intranasalen, subkutanen, oralen oder intramuskulären impfung verwendet wird oder durch andere Schleimhäute geimpft wird.

7. Die Impfstoffzubereitung nach einem der Ansprüche 4 bis 6, worin das Antigen abgeleitet ist von einem oder mehreren pathogenen Mikroorganismen, ausgewählt aus der Gruppe, bestehend aus Influenzavirus, Rotavirus, Maservirus, Rötelvirus, Mumpsvirus, AIDS-Virus, *Bordetella pertussis,* Diphterie-Bakterie, *Helicobacter pylori,* enterohämorrhagischen *Escherichia coli* (EHEC), *Chlamydien, Mycoplasma,* Malaria *Plasmodium,* Coccidium und Schistosomen.

8. Verwendung der Impfstoffzubereitung nach Anspruch 4 zur Herstellung eines Medikaments zur Behandlung von Krankheiten, umfassend die orale Verabreichung des Hilfsmittels in der Impfstoffzubereitung unabhängig von dem Antigenbestandteil.

9. Die Verwendung nach Anspruch 8, worin der Antigenbestandteil intranasal, subkutan, oral oder intramuskulär oder durch andere Schleimhäute geimpft wird.

## Revendications

1. Acide gras insaturé avec 18 atomes de carbone,
ou dérivé de celui-ci, qui a une structure trihydroxy-monoène, pour utilisation en tant qu'adjuvant.

2. Acide gras insaturé selon la revendication 1, dans lequel l'acide gras insaturé avec 18 atomes de carbone ou le dérivé de celui-ci qui a une structure trihydroxy-monoène est l'acide 9,12,13-trihydroxy-10E-octadécénoïque, ou un dérivé de celui-ci, dont la structure est là suivante: 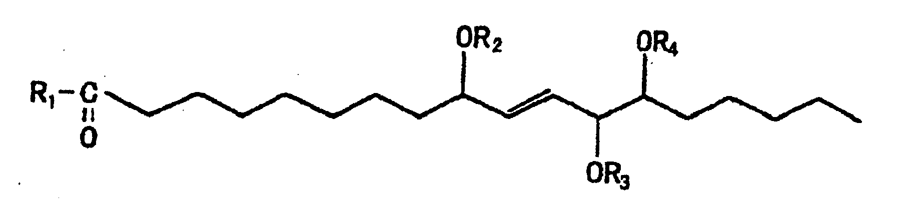 dans laquelle R₁ est choisi dans le groupe constitué par un groupe hydroxyle et un substituant comprenant une liaison de 1 ou 2 groupes alkyles ou groupes aryles à 1 atome d'oxygène, de soufre ou d'azote ; et R₂, R₃ et R₄ sont indépendamment choisis dans le groupe constitué par un hydrogène, un groupe alkyle et un groupe acyle et peuvent chacun être identiques ou différents.

3. Acide gras insaturé selon l'une quelconque des revendications 1 ou 2, dans lequel l'acide gras insaturé est un hydroxy-acide gras insaturé ou un dérivé de celui-ci préparé à partir d'une plante médicinale.

4. Préparation vaccinale comprenant un constituant antigénique et l'acide gras insaturé selon l'une quelconque des revendications 1 à 3 pour utilisation en tant qu'adjuvant, en tant que constituant.

5. Préparation vaccinale selon la revendication 4, dans laquelle l'adjuvant dans la préparation vaccinale est utilisé dans une inoculation orale indépendamment du constituant antigénique.

6. Préparation vaccinale selon la revendication 5, dans laquelle le constituant antigénique dans la préparation vaccinale est utilisé dans une inoculation intranasale, sous-cutanée, orale ou intramusculaire ou est inoculé par d'autres muqueuses.

7. Préparation vaccinale selon l'une quelconque des revendications 4 à 6, dans laquelle l'antigène est issu d'un ou plusieurs micro-organismes pathogènes choisis dans le groupe constitué par le virus de la grippe, un rotavirus, le virus de la rougeole, le virus de la rubéole, le virus des oreillons, le virus du SIDA, *Bordetella pertussis,* le bacille diphtérique, *Helicobacter pylori, Escherichia coli* entéro-hémorragique (EHEC), *Chlamydia, Mycoplasma, Plasmodium* du paludisme, coccidie et schistosome.

8. Utilisation de la préparation vaccinale selon la revendication 4 pour préparer un médicament destiné au traitement de maladies, comprenant l'administration orale de l'adjuvant dans la préparation vaccinale indépendamment du constituant antigénique.

9. Utilisation selon la revendication 8, dans laquelle le constituant antigénique est inoculé par voie intranasale, sous-cutanée, orale ou intramusculaire, ou par d'autres muqueuses.
